# FASCICULE DE BREVET EUROPEEN

(11) **EP 2 086 435 B1**
(45) Date de publication et mention de la délivrance du brevet: **18.12.2013**
(21) Numéro de dépôt: 07825262.4
(22) Date de dépôt: 04.10.2007
(51) Int. Cl.: A61B 17/70, A61B 17/80

(54) **Procédé de fabrication de plaques osseuses, notamment de plaques d'ostéosynthèse vertébrale**
Verfahren zur Herstellung von Knochenplatten, insbesondere Platten für die Wirbel-Osteosynthese
Method for production of bone plates, in particular of plates for vertebral osteosynthesis

(30) Priorité: 04.10.2006 FR 0608688; 04.10.2006 US 849013 P
(43) Date de publication de la demande: 12.08.2009
(73) Titulaire: Implants International Limited, Cleveland Thornaby-on-Tees TS17 9LZ (GB)
(72) Inventeur: EMMANUEL, Mohan, Station Town Durham TS28 5NA (GB)
(74) Mandataire: Jeannet, Olivier
(86) Numéro de dépôt international: PCT/IB2007/002938
(87) Numéro de publication internationale: WO 2008/041108

(56) Documents cités:
- EP-A1- 0 542 004
- US-A- 3 314 877
- US-A1- 2003 023 242
- US-A1- 2005 209 599
- US-B1- 6 398 783

## Description

La présente invention concerne un procédé de fabrication de plaques osseuses, notamment de plaques d'ostéosynthèse vertébrale.

Il est courant d'utiliser une plaque en titane ou alliage de titane pour maintenir deux vertèbres ou plus de deux vertèbres les unes par rapport aux autres, cette plaque venant se placer contre les corps vertébraux des vertèbres et étant fixée à ces derniers au moyen de vis.

Une telle plaque doit présenter une forme courbe dans le sens transversal pour s'adapter à la courbure des corps vertébraux et une forme courbe dans le sens longitudinal, lui permettant de s'adapter à la lordose des vertèbres. Elle comprend par ailleurs des trous permettant le passage au travers d'elle de ses vis de fixation, et peut comprendre des fentes, découpes, alésages, ouvertures, évidements ou autres.

Les plaques existantes sont à ce jour systématiquement réalisées à partir de blocs ou de bandes découpés dans des tôles laminées ou forgées de titane ou d'alliage de titane, ayant une épaisseur largement supérieure à l'épaisseur que doit avoir une plaque finie, les courbures précitées étant aménagées par usinage de cette bande dans la masse. Par exemple des bandes ayant des dimensions de 150 mm de long par 35 mm de large et 7 mm d'épaisseur, découpées transversalement dans des tôles, sont utilisées pour obtenir une série de plaques finies ayant une épaisseur de 2,5 mm.

Ce procédé de fabrication est considéré comme obligatoire pour obtenir une plaque à même de résister aux efforts répétés que subit cette plaque après implantation. Pour la même raison, il est considéré comme obligatoire qu'une telle plaque ait environ 2,5 mm d'épaisseur.

Le procédé traditionnel a pour inconvénient d'être long et coûteux à mettre en oeuvre, compte tenu du travail d'usinage à accomplir et de la quantité de matériau perdue.

De plus, ce procédé a pour inconvénient de créer des "zones de silence" ("dead zones" en anglais), c'est-à-dire des zones dans lesquelles l'intégrité du matériau est altérée par l'usinage agressif d'une quantité importante de ce matériau et par l'échauffement qui en résulte. La résistance à la fatigue des plaques s'en trouve affectée, rendant également nécessaire une épaisseur de celles-ci d'au moins 2,5 mm.

Les documents US 2005/0209599, EP 0 542 004, US 2003/0023242 et US 6,398,783 décrivent diverses plaques osseuses selon la technique antérieure et leurs procédés de fabrication.

La présente invention a pour but de remédier à l'ensemble de ces inconvénients.

Son objectif principal est donc de fournir un procédé de fabrication d'une plaque osseuse, notamment d'une plaque d'ostéosynthèse vertébrale, qui soit plus facile et moins onéreux à mettre en oeuvre que le procédé selon la technique antérieure, sans amoindrissement de la résistance de la plaque obtenue.

Un autre objectif de l'invention est de fournir un procédé permettant l'obtention de plaques ayant une épaisseur plus réduite que les plaques actuelles à résistance égale, notamment une épaisseur pouvant aller de 1,7 mm à 2 mm.

Un objectif supplémentaire de l'invention est de fournir un procédé évitant la création de "zones de silence" dans la plaque obtenue.

Pour atteindre au moins l'objectif principal ci-dessus, le procédé selon l'invention comprend les étapes suivantes :
a) utiliser une tôle de titane ou d'alliage de titane ayant une épaisseur de 1,7 à 2 mm ;
b) déterminer la direction des fibres de cette tôle ;
c) découper, parallèlement à la direction des fibres de cette tôle, des portions de tôle dimensionnées pour permettre l'obtention d'une pluralité de plaques osseuses, ces portions de tôle étant notamment sous forme de bandes ;
d) opérer dans un ordre quelconque les opérations d1, d2 et d3 ci-après :
   d1) mise en forme des portions de tôle, ou des plaques ou ébauches obtenues par l'opération d2, par roulage ou pliage à froid, pour aménager une courbure des portions de tôle ou des plaques ou des ébauches dans le sens transversal et/ou dans le sens longitudinal, afin d'obtenir une ou des courbures appropriées à la forme des vertèbres et/ou à la lordose des vertèbres auxquelles les plaques en cours de fabrication sont destinées ;
   d2) découpage, dans chaque portion de tôle, de plaques osseuses ou d'ébauches de plaques osseuses, de telle sorte qu'une direction longitudinale de ces plaques ou ébauches soit parallèle à la direction des fibres de la tôle ;
   d3) perçage et le cas échéant découpe et/ou usinage des portions de tôle, ou des plaques ou ébauches obtenues par l'opération d2 ;
e) finition des plaques osseuses ou des ébauches de plaques osseuses.

Le procédé selon l'invention consiste ainsi à déterminer la direction des fibres ("grain flow" en anglais) de la tôle, c'est-à-dire l'orientation des molécules du matériau résultant du laminage de la tôle, et à découper les plaques osseuses ou les ébauches de plaques osseuses de telle sorte que la longueur de ces plaques ou ébauches soit parallèle à la direction des fibres de la tôle, ces découpes intervenant avant ou après la mise en forme des bandes ou des plaques ou des ébauches.

La déposante en effet découvert que, dans le procédé traditionnel, les fibres de la tôle sont systématiquement orientées transversalement à la direction longitudinale des plaques osseuses obtenues, les bandes de tôles dans lesquelles ces plaques sont découpées ayant elles-mêmes été coupées transversalement à la direction des fibres de la tôle. Il en résulte la nécessité unanimement acceptée, pour obtenir la résistance adéquate, de réaliser des plaques osseuses dont les courbures sont obtenues par usinage et dont l'épaisseur minimale est de 2,5 mm.

Au contraire, le procédé selon l'invention permet d'obtenir des plaques avec les fibres de la tôle orientées dans le sens longitudinal des plaques obtenues, ce qui permet d'utiliser des tôles ayant une épaisseur limitée à 1,7 à 2 mm et de réaliser la mise en forme des portions de tôle ou des ébauches par des opérations relativement simples, notamment de roulage ou de pliage à froid ; les plaques osseuses obtenues ont une résistance comparable à celle des plaques de 2,5 mm d'épaisseur obtenues selon la technique antérieure, et n'impliquent pas la mise en oeuvre d'usinages long et onéreux à réaliser, susceptibles en outre de créer des altérations de l'intégrité du matériau ("zones de silence" ou "dead zones" en anglais).

La détermination de la direction des fibres de la tôle à l'étape b) peut être réalisée par une analyse macrographique de la surface de cette tôle.

Les découpes des portions de tôle ou des plaques ou ébauches aux étapes c) et d2) peuvent notamment être réalisées au moyen d'une machine à jet d'eau sous pression, notamment du type à commande numérique.

La découpe par jet d'eau est utilisée plutôt qu'un usinage ou qu'une découpe au laser parce qu'elle ne produit aucune chaleur de découpe qui créerait des particules susceptibles de s'effriter et modifierait défavorablement la structure des fibres autour du secteur découpé du matériau.

La courbure transversale réalisée à l'étape d1 peut notamment être générée par un rayon de 80 mm et la courbure longitudinale peut notamment être générée par un rayon de 300 à 400 mm.

De préférence, à l'étape d2, les ébauches découpées comprennent des pattes saillantes permettant leur immobilisation sur une machine appropriée en vue de la réalisation de tout ou partie des opérations de l'étape d3) et de l'étape e) ; les opérations de finition de l'étape e) incluent alors l'élimination de ces pattes saillantes après réalisation des autres opérations de finition. Ladite machine appropriée peut notamment être une machine de façonnage à quatre ou cinq axes (les trois axes cartésiens et un ou deux axes de rotation).

Selon une forme possible de mise en oeuvre de l'invention, le procédé comprend les étapes suivantes :
- étape a) ;
- étape b) ;
- étape c) ;
- étape d1) : mise en forme des portions de tôle uniquement dans le sens transversal ;
- étape d2) ;
- étape d3) ;
- étape d4) : mise en forme des plaques ou ébauches obtenues à l'étape d2), pour aménager une courbure dans le sens longitudinal, appropriée à la lordose des vertèbres auxquelles les plaques osseuses sont destinées ;
- étape e).

Les opérations de l'étape d3) (perçages et autres) et d4) (courbure longitudinale) sont donc réalisées sur des plaques ou ébauches de plaques osseuses découpées à l'étape d2).

Selon une autre forme possible de mise en oeuvre de l'invention, le procédé comprend les étapes suivantes :
- étape a) ;
- étape b) ;
- étape c) ;
- étape d1) : mise en forme des portions de tôle dans le sens transversal et dans le sens longitudinal ;
- étape d3) ;
- étape d2) ;
- étape e).

Les opérations de l'étape d1) (courbures transversale et longitudinale) et d3) (perçages et autres) sont donc réalisés sur les portions de tôle obtenues à l'étape c).

Le procédé peut comprendre, après l'étape d1 ou d4, la réalisation d'une analyse macrographique de la surface d'au moins un des côtés de ladite portion de tôle, ou desdites plaques ou ébauches.

Cette analyse permet de s'assurer qu'aucun défaut de surface ou fissure n'a été crée durant ces étapes d1) ou d4).

L'invention sera bien comprise, et d'autres caractéristiques et avantages de celle-ci apparaîtront, en référence au dessin schématique annexé, représentant, à titre d'exemples non limitatifs, des portions de tôle au cours de deux mises en oeuvre possibles du procédé qu'elle concerne.
Les figures 1 à 4 sont des vues très schématiques de portions de tôle selon une première mise en oeuvre du procédé, et
les figures 5 à 8 sont des vues très schématiques de portions de tôle selon une deuxième mise en oeuvre du procédé.

Par simplification, les parties ou éléments qui se retrouvent de manière identique ou similaire dans ces deux mises en oeuvre seront identifiés par les mêmes références numériques et ne seront pas à nouveau décrits.

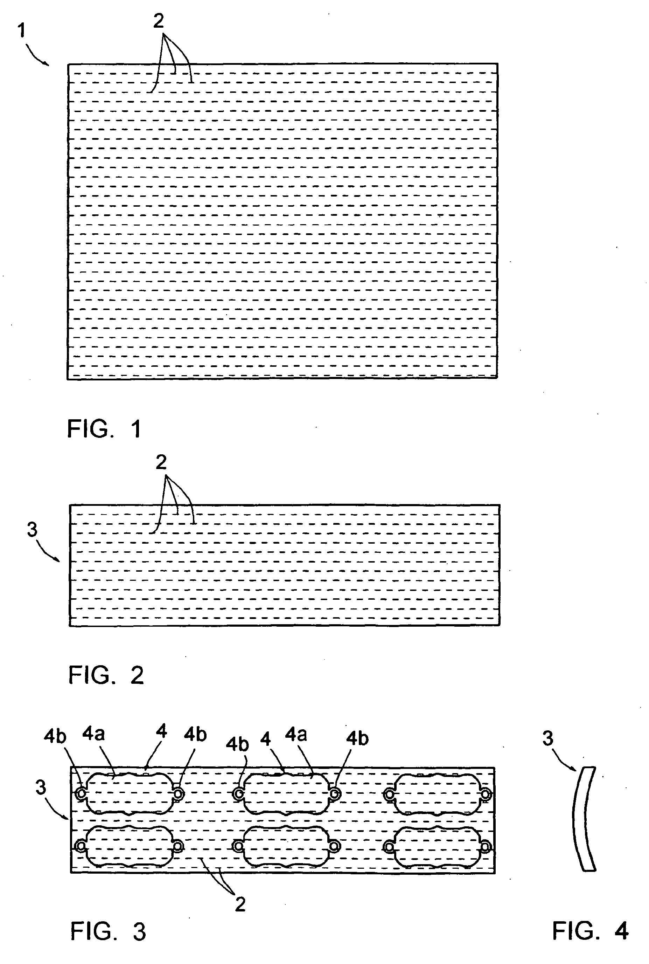

La figure 1 représente une tôle 1 de titane ou d'alliage de titane ayant une épaisseur de 1,7 à 2 mm, dont les fibres, schématisées par des lignes pointillées 2, sont orientées dans une direction déterminée ("grain flow" en anglais). Cette direction est celle de l'orientation des molécules du matériau qui résulte du laminage de la tôle 1.
La figure 2 montre une portion 3 de la tôle 1 dimensionnée pour permettre l'obtention d'une pluralité de plaques osseuses, ces plaques osseuses étant des plaques d'ostéosynthèse vertébrale. La portion de tôle 3 a été découpée parallèlement à la direction des fibres de la tôle 1 ; elle peut par exemple avoir 160 mm de long et 40 mm de large.
La figure 3 montre des ébauches 4 de plaques osseuses telles qu'elles vont être découpées dans la portion de tôle 3 ; elles comprennent une partie centrale 4a destinée à former la plaque d'ostéosynthèse proprement dite et, à chaque extrémité longitudinale de cette partie centrale, une patte saillante 4b percée d'un trou. Ces pattes saillantes 4b permettent l'immobilisation des ébauches 4 sur une machine appropriée en vue de la réalisation de tout ou partie d'opérations de perçage, et le cas échéant de découpe et/ou d'usinage limité des ébauches 4, et d'opérations de finition des plaques osseuses. Ces opérations de finition incluent notamment l'élimination des pattes saillantes 4b.

Il est essentiel de remarquer que les ébauches 4 sont découpées de telle sorte que la direction longitudinale de ces ébauches est parallèle à la direction des fibres de la portion de tôle 3.

La figure 4 montre que la portion de tôle 3 a été courbée transversalement avant découpes des ébauches 4, notamment par roulage ou pliage à froid, de manière à ce que ces ébauches 4 présentent, après découpe, une courbure appropriée à la forme des corps vertébraux des vertèbres dont elles sont destinées à assurer la fixation mutuelle. Le rayon générant cette courbure peut notamment être de 80 mm.

Le procédé correspondant aux figures 1 à 4 comprend les étapes suivantes :
- étape a) : utiliser une tôle 1 de titane ou d'alliage de titane ayant une épaisseur de 1,7 à 2 mm ;
- étape b) : déterminer la direction des fibres de cette tôle, par une analyse macrographique de la surface de la tôle 1 ;
- étape c) : découper, au moyen d'une machine à jet d'eau sous pression à commande numérique, des portions 3 de tôle 1, parallèlement à la direction des fibres de cette tôle 1 ;
- étape d1) : mise en forme par roulage ou pliage à froid des portions 3 de tôle 1 dans le sens transversal, comme montré sur la figure 4 ;
- étape d2) : découpage, au moyen d'une machine à jet d'eau sous pression à commande numérique, des ébauches 4 dans chaque portion 3 de tôle 1, de telle sorte qu'une direction longitudinale de ces ébauches 4 soit parallèle à la direction des fibres de la tôle 1 ;
- étape d3) : mise en place des ébauches 4, au moyen des pattes 4b, dans une machine de façonnage à quatre ou cinq axes (les trois axes cartésiens et un ou deux axes de rotation) puis perçage, et le cas échéant découpe et/ou usinage limité des ébauches 4 ;
- étape d4) : mise en forme par roulage ou pliage à froid des ébauches 4 afin de leur conférer une courbure dans le sens longitudinal leur permettant d'être adaptées à la lordose anatomique des vertèbres auxquelles les plaques osseuses sont destinées ; cette courbure est générée par un rayon pouvant aller de 300 à 400 mm ;
- étape e) : opérations de finition de ces plaques et obtention des plaques osseuses par élimination des pattes 4b.

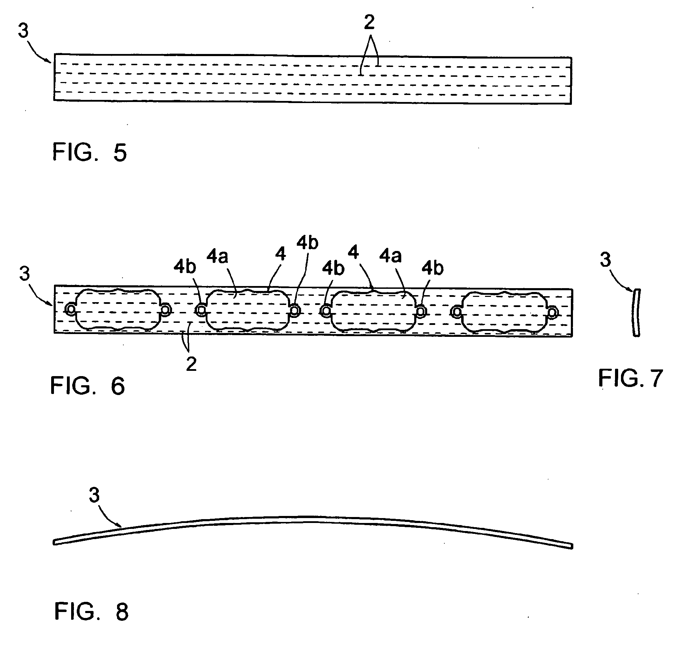

Les figures 5 à 7 correspondent respectivement aux figures 2 à 4, montrant une portion 3 de tôle 1 telle que précitée, qui est courbée puis dans laquelle sont découpées les ébauches 4.

Dans ce cas, cependant :
- la largeur de la portion 3 est inférieure à celle de la portion 3 du cas précédent, permettant uniquement l'obtention d'une rangée d'ébauches 4.
- la portion 3 est pliée longitudinalement avant la découpe des ébauches 4, ainsi que le montre la figure 8.

Le procédé correspondant aux figures 5 à 8 comprend les étapes suivantes :
- étape a) telle que précitée ;
- étape b) telle que précitée ;
- étape c) telle que précitée ;
- étape d1): mise en forme des portions de tôle 3 dans le sens transversal et dans le sens longitudinal ;
- étape d3) : mise en place des portions de tôle 3 dans une machine de façonnage à quatre ou cinq axes (les trois axes cartésiens et un ou deux axes de rotation) puis perçage, et le cas échéant découpe et/ou usinage limité aux endroits adéquat ;
- étape d2) retrait des portions de tôle 3 de ladite machine et découpe des ébauches 4 telle que précitée ;
- étape e) telle que précitée.

Les opérations de l'étape d1) (courbures transversale et longitudinale) et d3) (perçages et autres) sont donc réalisés sur les portions de tôle obtenues à l'étape c).

L'une ou l'autre des mises en oeuvre précitées du procédé peut comprendre, après l'étape d1 ou d4, la réalisation d'une analyse macrographique de la surface d'au moins un des côtés de la portion de tôle 3, ou desdites ébauches 4 afin de s'assurer qu'aucun défaut de surface ou fissure n'a été crée durant ces étapes d1) ou d4).

L'invention fournit ainsi un procédé de fabrication de plaques osseuses, notamment de plaques d'ostéosynthèse vertébrale, ayant pour avantages déterminants :
- d'être plus facile et moins onéreux à mettre en oeuvre que le procédé selon la technique antérieure, sans amoindrissement de la résistance de la plaque obtenue ;
- de permettre l'obtention de plaques ayant une épaisseur plus réduite que les plaques actuelles à résistance égale, notamment une épaisseur pouvant aller de 1,7 mm à 2 mm ;
- d'éviter la création de "zones de silence" dans la plaque obtenue.

Il va de soi que l'invention n'est pas limitée à la forme de réalisation décrite ci-dessus à titre d'exemple mais qu'elle s'étend à toutes les formes de réalisations couvertes par les revendications ci-annexées.

## Revendications

1. Procédé de fabrication de plaques osseuses, notamment de plaques d'ostéosynthèse vertébrale, **caractérisé en ce qu'**il comprend les étapes suivantes :
a) utiliser une tôle (1) de titane ou d'alliage de titane ayant une épaisseur de 1,7 à 2 mm;
b) déterminer la direction des fibres de cette tôle (1) ;
c) découper, parallèlement à la direction des fibres de cette tôle (1), des portions (3) de tôle (1) dimensionnées pour permettre l'obtention d'une pluralité de plaques osseuses, ces portions (3) de tôle étant notamment sous forme de bandes ;
d) opérer dans un ordre quelconque les opérations d1, d2 et d3 ci-après :
d1) mise en forme des portions (3) de tôle, ou des plaques ou ébauches (4) obtenues par l'opération d2, par roulage ou pliage à froid, pour aménager une courbure des portions (3) de tôle ou des plaques ou des ébauches (4) dans le sens transversal et/ou dans le sens longitudinal, afin d'obtenir une ou des courbures appropriées à la forme des vertèbres et/ou à la lordose des vertèbres auxquelles les plaques en cours de fabrication sont destinées ;
d2) découpage, dans chaque portion (3) de tôle, de plaques osseuses ou d'ébauches (4) de plaques osseuses, de telle sorte qu'une direction longitudinale de ces plaques ou ébauches (4) soit parallèle à la direction des fibres de la tôle (1) ;
d3) perçage et le cas échéant découpe et/ou usinage des portions (3) de tôle, ou des plaques ou ébauches (4) obtenues par l'opération d2 ;
e) finition des plaques osseuses ou des ébauches (4) de plaques osseuses.

2. Procédé selon la revendication 1, **caractérisé en ce que** la détermination de la direction des fibres de la tôle (1) est réalisée par une analyse macrographique de la surface de cette tôle (1).

3. Procédé selon la revendication 1 ou la revendication 2, **caractérisé en ce que** les découpes des portions (3) de tôle ou des plaques ou ébauches (4) aux étapes c) et d2) sont réalisées au moyen d'une machine à jet d'eau sous pression, notamment du type à commande numérique.

4. Procédé selon l'une des revendications 1 à 3, **caractérisé en ce que** la courbure transversale réalisée à l'étape d1 est générée par un rayon de 80 mm et la courbure longitudinale est générée par un rayon de 300 à 400 mm.

5. Procédé selon l'une des revendications 1 à 4, **caractérisé en ce que**, à l'étape d2, les ébauches (4) découpées comprennent des pattes saillantes (4b) permettant leur immobilisation sur une machine appropriée en vue de la réalisation de tout ou partie des opérations de l'étape d3) et de l'étape e) ; les opérations de finition de l'étape e) incluent alors l'élimination de ces pattes saillantes (4b) après réalisation des autres opérations de finition.

6. Procédé selon la revendication 5, **caractérisé en ce que** ladite machine appropriée est une machine de façonnage à quatre ou cinq axes (les trois axes cartésiens et un ou deux axes de rotation).

7. Procédé selon l'une des revendications 1 à 6, **caractérisé en ce qu'**il comprend les étapes suivantes :
- étape a) ;
- étape b) ;
- étape c) ;
- étape d1) : mise en forme des portions (3) de tôle uniquement dans le sens transversal ;
- étape d2) ;
- étape d3) ;
- étape d4) : mise en forme des plaques ou ébauches (4) obtenues à l'étape d2), pour aménager une courbure dans le sens longitudinal, appropriée à la lordose des vertèbres auxquelles les plaques osseuses sont destinées ;
- étape e).

8. Procédé selon l'une des revendications 1 à 6, **caractérisé en ce qu'**il comprend les étapes suivantes :
- étape a) ;
- étape b) ;
- étape c) ;
- étape d1): mise en forme des portions (3) de tôle dans le sens transversal et dans le sens longitudinal ;
- étape d3) ;
- étape d2) ;
- étape e).

9. Procédé selon l'une des revendications 1 à 8, **caractérisé en ce qu'**il comprend, après l'étape d1 ou d4, la réalisation d'une analyse macrographique de la surface d'au moins un des côtés de ladite portion (3) de tôle, ou desdites plaques ou ébauches (4).

## Patentansprüche

1. Verfahren zur Herstellung von Knochenplatten, insbesondere Platten für die Osteosynthese der Wirbelsäule, **dadurch gekennzeichnet, dass** es die folgenden Schritte umfasst:
a) Verwendung eines Bleches (1) aus Titan oder Titanlegierung mit einer Dicke von 1 mm 7-2
b) Bestimmen der Richtung der Fasern dieses Bleches (1);
c) schneiden, parallel zur Faserrichtung der Bleches (1) Abschnitte (3) der Folie (1) zu ermöglichen, Erhalten einer Vielzahl von Knochenplatten dimensioniert, wobei die Abschnitte (3) insbesondere in Form von Platten Band;
d) Betrieb in beliebiger Reihenfolge die Schritte von d1, d2 und d3:
d1) formgebenden die Abschnitte (3) der Bleche oder Rohlinge oder (4) durch den Betrieb d2 erhalten, insbesondere durch Walzen oder Kaltformen, um eine Krümmung der Abschnitte (3) aus Blech oder Platten oder Zuschnitte (4) in der Querrichtung und / oder in Längsrichtung aufzunehmen, um eine geeignete Krümmungen oder die Form der Wirbel und / oder der Lendenwirbel der Platten, die während der Herstellung erhalten sollen;
d2) Schneiden in jedem Abschnitt (3) aus Blech, oder Knochenplatten oder Zuschnitte (4) der Knochenplatte so dass eine Längsrichtung der Platten oder der Zuschnitte (4) parallel zu der Faserrichtung der Bahn (1) ist;
d3) Bohren und gegebenenfalls Schneiden und / oder Bearbeitung der Abschnitte (3) der Bleche oder Rohlinge oder (4) durch den Betrieb d2 erhalten;
e) Die Endfertigung von Knochenplatten oder Zuschnitte (4) der Knochenplatte.

2. Verfahren nach Anspruch 1, **dadurch gekennzeichnet, dass** die Bestimmung der Richtung der Fasern von der Folie (1) durch eine Oberflächenanalyse makrographische dieser Folie (1) gebildet ist.

3. Verfahren nach Anspruch 1 oder Anspruch 2, **dadurch gekennzeichnet, daß** die Abschnitte der Zuschnitte (3) oder Blechen oder Platinen (4) die Schritte c) und d2) aus mittels eines Jet-Anlage durchgeführt von Wasser unter Druck, insbesondere von eine numerischen Steuerung.

4. Verfahren nach einem der Ansprüche 1 bis 3, **dadurch gekennzeichnet, daß** die transversale Krümmung in Schritt d1 erzeugt durch einen Radius von 80 mm und die longitudinale Krümmung mit einem Radius von 300 bis 400 mm erzeugt wird.

5. Verfahren nach einem der Ansprüche 1 bis 4, **dadurch gekennzeichnet, dass** in Schritt d2 weisen die Platinen (4) geschnitten vorspringenden Zungen (4b) für die Befestigung eines geeigneten Maschine zur Durchführung alle oder einen Teil der Operationen Schritt d3) und Schritt e), Nachbehandlungen von Schritt e) anschließendes Entfernen Dazu gehören vorspringenden Zungen (4b) nach Abschluss der anderen Nachbehandlungen.

6. Verfahren nach Anspruch 5, **dadurch gekennzeichnet, dass** die geeignete Maschine ist eine Arbeitsmaschine mit vier oder fünf Stifte (drei kartesischen Achsen und eine oder zwei Drehachsen)

7. Verfahren nach einem der Ansprüche 1 bis 6, **dadurch gekennzeichnet, dass** es die folgenden Schritte umfasst:
- Schritt a);
- Schritt b);
- Schritt c);
- Schritt d1), formgebenden Abschnitte (3) des Blattes nur in Querrichtung - Schritt d2);
- Schritt d3);
- Schritt d4): formgebenden Platten oder Zuschnitte (4) in Schritt d2) erhaltenen, um eine Biegung in Längsrichtung, die für die Lendenwirbel Knochenplatten, die dazu bestimmt unterzubringen
- Schritt e).

8. Verfahren nach einem der Ansprüche 1 bis 6, **dadurch gekennzeichnet, dass** es die folgenden Schritte umfasst:
- Schritt a);
- Schritt b)
- Schritt c);
- Schritt d1), formgebenden die Abschnitte (3) der Folie in der Querrichtung und in der Längsrichtung;
- Schritt d3);
- Schritt d2),
- den Schritt e).

9. Verfahren nach einem der Ansprüche 1 bis 8, **dadurch gekennzeichnet, dass** es nach dem Schritt d1 und d4, Durchführen einer Oberflächenanalyse makrographische wenigstens einer Seite des Abschnitts (3) aus Blech, wobei die Platte oder Platten oder Zuschnitte (4).

## Claims

1. Method for manufacturing bone plates, in particular plates for vertebral osteosynthesis, **characterized in that** it comprises the following steps:
a) using a sheet (1) of titanium or titanium alloy having a thickness of 1,7-2 mm;
b) determining the direction of the grain flow of this sheet (1);
c) cutting out portions (3) of sheet (1), parallel to the direction of the grain flow of this sheet (1), sized so as to make it possible to obtain a plurality of bone plates, these portions (3) of sheet in particular being in the form of strips;
d) performing, in any order, operations d1, d2 and d3 below:
d1) shaping the portions (3) of sheet, or plates or blanks (4) obtained by operation d2, by rolling or cold bending, in order to form a curvature of the portions (3) of sheet or of the plates or blanks (4) in the transverse direction and/or the longitudinal direction, in order to obtain one or several curvatures suited to the shape of the vertebrae and/or the lordosis of the vertebrae for which the plates being manufactured are intended;
d2) cutting out, in each portion (3) of sheet, bone plates or bone plate blanks (4), such that a longitudinal direction of these plates or blanks (4) is parallel to the direction of the grain flow of the sheet;
d3) piercing and, if necessary, cutting out or machining portions (3) of sheet, or plates or blanks (4) obtained by operation d2;
e) finishing the plates or bone plate blanks (4).

2. Method according to claim 1, **characterized in that** the determination of the grain flow of the sheet (1) is done through a macrographic analysis of the surface of this sheet (1).

3. Method according to claim 1 or claim 2, **characterized in that** the cut-outs of the portions (3) of sheet or of the plates or blanks (4) in steps c) and d2) are done using a pressurized water jet machine, in particular of the numerical control type.

4. Method according to anyone of claims 1 to 3, **characterized in that** the transverse curvature produced in step d1 is generated by a radius of 80 mm and the longitudinal curvature is generated by a radius of 300 to 400 mm.

5. Method according to anyone of claims 1 to 4, **characterized in that**, in step d2, the cutout blanks (4) comprise protruding legs (4b) allowing them to be immobilized on a suitable machine in order to perform all or some of the operations of step d3) and step e); the finishing operations of step e) then include the elimination of these protruding legs (4b) after performing the other finishing operations.

6. Method according to claim 5, **characterized in that** said suitable machine is a four- or five-axis shaping machine (the three Cartesian axes and one or two axes of rotation).

7. Method according to anyone of claims 1 to 6, **characterized in that** comprises the following steps:
- step a);
- step b);
- step c);
- step d1): formation of the portions (3) of sheet only in the transverse direction;
- step d2);
- step d3);
- step d4): shaping of the plates or blanks (4) obtained in step d2) in order to form a curve in the longitudinal direction, suitable for the lordosis of the vertebrae for which the bone plates are intended;
- step e).

8. Method according to anyone of claims 1 to 6, **characterized in that** comprises the following steps:
- step a);
- step b);
- step c);
- step d1): shaping of the portions (3) of sheet in the transverse direction and in the longitudinal direction;
- step d3);
- step d2);
- step e).

9. Method according to anyone of claims 1 to 8, **characterized in that** it comprises, after step d1 or d4, the performance of a macrographic analysis of the surface of at least one of the sides of said portion (3) of sheet, or of said plates or blanks (4).
